# EUROPEAN PATENT APPLICATION

(11) **EP 2 913 053 A1**
(43) Date of publication of application: **02.09.2015**
(21) Application number: 13848655.0
(22) Date of filing: 22.10.2013
(51) Int. Cl.: A61K 31/426, A61P 13/04, A61P 13/12, A61P 35/00, A61P 39/00

(54) **THERAPEUTIC OR PROPHYLACTIC AGENT FOR TUMOR LYSIS SYNDROME**

(30) Priority: 23.10.2012 JP 2012233806; 10.01.2013 JP 2013002913
(71) Applicant: Teijin Pharma Limited, Tokyo 100-0013 (JP)
(72) Inventor: UEDA, Takanori, Yoshida-gun Fukui 910-1193 (JP); YAMAUCHI, Takahiro, Yoshida-gun Fukui 910-1193 (JP); MORITA, Mihoko, Yoshida-gun Fukui 910-1193 (JP); HORIUCHI, Hideki, Tokyo 100-0013 (JP)
(74) Representative: Carpmaels & Ransford LLP
(86) International application number: PCT/JP2013/078571
(87) International publication number: WO 2014/065275

(57) **Abstract**

The purpose of the present invention is to provide a novel therapeutic or prophylactic agent for tumor lysis syndrome. The present invention is a therapeutic or prophylactic agent for tumor lysis syndrome, which comprises a 2-phenylthiazole compound represented by formula (I) or a pharmaceutically acceptable salt thereof as an active ingredient.

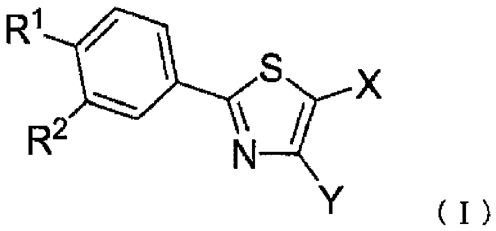

## Description

### [TECHNICAL FIELD]

The present invention relates to therapeutics and prophylactics for tumor lysis syndrome comprising as an active ingredient a 2-phenylthiazole compound represented by formula (I) or a pharmaceutically acceptable salt thereof.

### [BACKGROUND ART]

Tumor lysis syndrome (hereinafter sometimes referred to as "TLS") is a metabolic disorder caused by the massive and abrupt release of cellular components into the blood when tumor cells are killed by introduction of chemotherapy, radiotherapy, etc., or when spontaneous lysis of tumors occurs. When tumor cells die rapidly, nucleic acids, proteins, potassium, phosphorus, and the like flow into the blood at the same time, which as a result causes hyperuricemia, hyperkalemia, hyperphosphatemia, hypocalcemia, uremia, and the like.

Among others, hyperuricemia results from the metabolism of the purine bodies included in nucleic acids and the like in cells by xanthine oxidase followed by the production of uric acid via hypoxanthine and xanthine; high concentrations of urates which exceed their solubility limits in the body result in crystal deposition in the kidneys and the urinary tract, which induces renal dysfunction, or even acute uric acid nephropathy, i.e., acute renal failure, leading to death. Since the symptoms are usually observed 12 to 72 hours after starting a therapy, it is necessary to properly assess patients for risk, classify them into high risk, intermediate risk, and low risk groups, and undertake sufficient preventive measures and close monitoring, and, when it occurs, it is necessary to perform an appropriate treatment in its early stages.

For patients with high probabilities of developing tumor lysis syndrome, a urine volume is increased by pre-supplying sufficient fluid in order to prevent acute uric acid nephropathy, and, since crystals are prone to develop when urine is acidic, urinary alkalization is attempted. Besides, in order to lower uric acid levels, there is performed a therapeutic method in which uric acid production is inhibited by means of allopurinol, a xanthine oxidase inhibitor, or a therapeutic method in which uric acid is degraded by means of rasburicase, a urate oxidizing enzyme. Note that the xanthine oxidase inhibitor is an agent that has been applied clinically as a therapeutic agent for gout and hyperuricemia.

However, it is known that since the mechanism of action of the xanthine oxidase inhibitor allopurinol is through inhibition of the formation of uric acid, it is ineffective when uric acid levels are already high. In addition, since it takes several days for the uric acid level-lowering effect of allopurinol to appear, the start of chemotherapy to lyse tumors may have to be delayed when the agent is used prophylactically (Non-Patent Document 1). Further, the inhibition of xanthine oxidase by allopurinol may result in increased urinary concentrations of xanthine and its deposition in the urinary tract and cause acute obstructive uropathy to develop (Non-Patent Document 1). Because of these limitations, for cases at intermediate risk for tumor lysis syndrome, allopurinol is used prophylactically from not less than 12 hours before the start of chemotherapy, while increasing a urine volume by infusing sufficient fluid (Non-Patent Document 2). As for the dosage regimen of allopurinol, it is recommended to be administered at 50 to 100 mg/m² or 10 mg/kg/day divided every 8 hours (Non-Patent Document 1).

On the other hand, in the treatment or prevention of tumor lysis syndrome, the usefulness of rasburicase, which is a recombinant urate oxidase having the effect of decomposing uric acid in the blood, has recently been demonstrated (Non-Patent Document 3). However, care should be taken, as it can cause hypersensitivity, hemolytic anemia, and methemoglobinemia. In addition, because its dose frequency is limited as antibodies develop in the body of patients to whom it has been administered, and because of its high manufacturing cost and high price compared with small molecule agents, it is not used frequently, and it is recommended to be used mainly in patients at high risk for tumor lysis syndrome (Non-Patent Document 1). For intermediate-risk cases, administration of rasburicase is recommended to be performed therapeutically when hyperuricemia is already present, or when the risk increases, such as when hyperuricemia occurs during chemotherapy (Non-Patent Document 2).

2-[3-cyano-4-(2-methylpropoxy)phenyl]-4-methylthiazole-5-carboxylic acid (generic name: febuxostat), which is used in the present invention, is known to have the effect of lowering uric acid levels through its xanthine oxidase inhibitory effect, the relationship between dose level and efficacy thereof has been investigated, and it has been found to be a therapeutic agent for hyperuricemia and gout (Non-Patent Documents 4 to 6). Febuxostat, like allopurinol, is a xanthine oxidase inhibitor.

### [RELATED ART DOCUMENTS]

### [NON-PATENT DOCUMENTS]

[Non-Patent Document 1] Journal of Clinical Oncology, vol.26, No.16, 2008, 2767-2778.
[Non-Patent Document 2] Clinical Practice Guidelines for Pediatric Leukemia/Lymphoma 2011 [Shoni Hakketsubyo Rinpashu no Shinryo Gaidorain 2011-nenban].
[Non-Patent Document 3] Journal of Clinical Oncology, vol.19, No.3, 2001, 697-704.
[Non-Patent Document 4] Journal of Clinical Rheumatology, vol.17, No.4, 2011, S35-S43.
[Non-Patent Document 5] Journal of Clinical Rheumatology, vol.17, No.4, 2011, S44-S49.
[Non-Patent Document 6] Journal of Clinical Rheumatology, vol.17, No.4, 2011, S50-S56.

### [SUMMARY OF THE INVENTION]

### [PROBLEMS TO BE SOLVED BY THE INVENTION]

It is an object of the present invention to provide a novel therapeutic or prophylactic agent for tumor lysis syndrome.

### [MEANS OF SOLVING THE PROBLEMS]

When administering the compound in the present invention to a high-risk patient who already presented hyperuricemia due to tumor lysis, the present inventors found that blood uric acid levels were lowered dramatically, and at the same time, renal function was also improved dramatically.

In addition, when prophylactically administering a low dose of the compound in the present invention to hematopoietic tumor patients at low or intermediate risk for tumor lysis syndrome and subsequently performing chemotherapy on tumor cells, the present inventors found that, in all cases, it maintained blood uric acid levels within or lowered them to within a normal range (7.5 mg/dL or less) and also improved renal function. Furthermore, in a severely ill patient with a particularly elevated white blood cell count (WBC) among these patients, the effect of dramatically lowering urinary excretion levels of uric acid was also found.

That is, the present invention is as follows.
(1) A therapeutic or prophylactic agent for tumor lysis syndrome, comprising as an active ingredient a 2-phenylthiazole compound represented by the following formula (I):

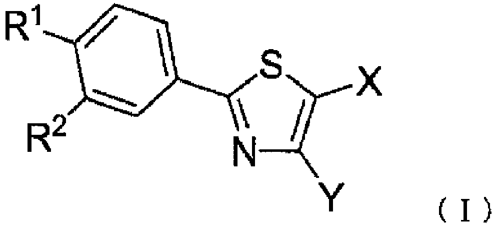

   wherein:
   R¹ represents a C₁-C₈ alkoxy group, a morpholino group, a 4-methylpiperazin-1-yl group, or a piperidino group;
   R² represents a nitro group or a cyano group;
   X represents a carboxyl group or a C₂-C₇ alkoxycarbonyl group; and
   Y represents a hydrogen atom or a C₁-C₆ alkyl group;
   or a pharmaceutically acceptable salt thereof.
(2) The therapeutic or prophylactic agent according to (1), wherein the tumor lysis syndrome is a tumor lysis syndrome in a high-risk patient.
(3) The therapeutic or prophylactic agent according to (1), wherein the tumor lysis syndrome is a tumor lysis syndrome in an intermediate-risk patient.
(4) The therapeutic or prophylactic agent according to (1), wherein the tumor lysis syndrome is a tumor lysis syndrome in a low-risk patient.
(5) The therapeutic or prophylactic agent according to any one of (1) to (4), wherein the tumor lysis syndrome is a tumor lysis syndrome involves hyperuricemia caused by chemotherapy or radiotherapy for malignant tumor.
(6) The therapeutic or prophylactic agent according to any one of (1) to (4), wherein the tumor lysis syndrome is a tumor lysis syndrome which occurs without being caused by antitumor therapy and in which hyperuricemia and reduced renal function are occurred.
(7) The therapeutic or prophylactic agent according to any one of (1) to (6), wherein the tumor lysis syndrome is a tumor lysis syndrome in a case where hyperuricemia is presented before implementation of antitumor therapy.
(8) The therapeutic or prophylactic agent according to any one of (1) to (7), wherein the tumor lysis syndrome is a tumor lysis syndrome in a case where renal function is reduced before implementation of antitumor therapy.
(9) The therapeutic or prophylactic agent according to any one of (1) to (8), characterized in that the 2-phenylthiazole compound represented by the formula (I), or a pharmaceutically acceptable salt thereof, is administered at 0.5 to 1000 mg per day, 1 to 7 days/week.
(10) The therapeutic or prophylactic agent according to any one of (1) to (9), which can lower blood uric acid levels following implementation of antitumor therapy to within normal levels or maintain them within the normal levels.
(11) The therapeutic or prophylactic agent according to any one of (1) to (10), which can improve renal function following implementation of antitumor therapy, compared with that before start of the antitumor therapy, or keep it normal.
(12) The therapeutic or prophylactic agent according to any one of (1) to (11), which can lower urinary excretion levels of uric acid following implementation of antitumor therapy to within normal levels or maintain them within the normal levels.
(13) The therapeutic or prophylactic agent according to any one of (1) to (12), wherein the 2-phenylthiazole compound represented by the above formula (I) is 2-[3-cyano-4-(2-methylpropoxy) phenyl]-4-methylthiazole-5-carboxylic acid or a pharmaceutically acceptable salt thereof.

### [EFFECTS OF THE INVENTION]

According to the present invention, it is possible to treat or prevent tumor lysis syndrome by administering the 2-phenylthiazole compound represented by the formula (I) or a pharmaceutically acceptable salt thereof.

In addition, the compounds in the present invention can sufficiently lower blood uric acid levels and thus are possible to administer to intermediate-risk patients and high-risk patients not only as therapeutic agents, but also as prophylactic agents, for tumor lysis syndrome.

Furthermore, the present invention can not only sufficiently lower blood uric acid levels and maintain them within a normal range, but also lower urinary excretion levels of uric acid and maintain them within a normal range, and can improve renal function or prevent it from deteriorating.

### [BRIEF DESCRIPTION OF THE DRAWINGS]

[Figure 1] Figure 1 shows time course of blood uric acid level (UA), serum creatinine level (Cre), and estimated glomerular filtration rate (eGFR) in a high-risk tumor lysis syndrome patient following administration of febuxostat to the patient in Example 1.
[Figure 2] Figure 2 shows changes in serum uric acid level (UA) before and after the administration of febuxostat from just before or the day before chemotherapy was performed on patients with hematological malignancies at low or intermediate risk for tumor lysis syndrome in Example 2.
[Figure 3] Figure 3 shows changes in estimated glomerular filtration rate (eGFR) before and after the administration of febuxostat from just before or the day before chemotherapy was performed on patients with hematological malignancies at low or intermediate risk for tumor lysis syndrome in Example 2.
[Figure 4] Figure 4 shows changes in urinary excretion level of uric acid (UA) before and after the administration of febuxostat from just before or the day before a chemotherapeutic agent was performed on patients with hematological malignancies at low or intermediate risk for tumor lysis syndrome in Example 2.

### [MODE FOR CARRYING OUT THE INVENTION]

The 2-phenylthiazole compounds in the present invention represented by the following formula (I):

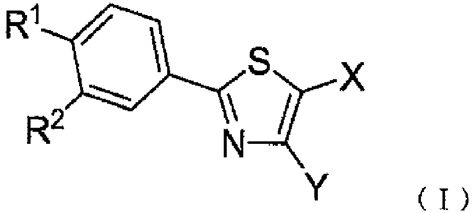

wherein:
R¹ represents a C₁-C₈ alkoxy group, a morpholino group, a 4-methylpiperazin-1-yl group, or a piperidino group;
R² represents a nitro group or a cyano group;
X represents a carboxyl group or a C₂-C₇ alkoxycarbonyl group; and
Y represents a hydrogen atom or a C₁-C₆ alkyl group;
or pharmaceutically acceptable salts thereof, include, for example, 2-[3-cyano-4-(2-methylpropoxy) phenyl]-4-methylthiazole-5-carboxylic acid. Further, the compounds represented by the formula (I) can be produced by known methods, such as the method described in International Publication No. WO92/09279.

The "C₁-C₈ alkoxy group" in R¹ of the above formula (I) means a group consisting of a C₁-C₈ linear or branched alkyl group, such as, for example, methyl, ethyl, n-propyl, n-butyl, n-pentyl, n-hexyl, n-heptyl, n-octyl, isopropyl, isobutyl, sec-butyl, tert-butyl, isopentyl, neopentyl, tert-pentyl, isohexyl, 2-methylpentyl, and 1-ethylbutyl groups, and of an oxy group, suitable specific examples of which include methoxy, ethoxy, n-propyloxy, n-butyloxy, isopropyloxy, isobutyloxy, sec-butyloxy, tert-butyloxy, isopentyloxy, neopentyloxy groups, and the like. More preferred examples include isobutyloxy group. Preferred groups for R¹ are C₁-C₈ alkoxy groups, and a more preferred group is an isobutyloxy group.

A preferred group for R² is a cyano group.

The "C₂-C₇ alkoxycarbonyl group" in X of the above formula (I) means a group consisting of a C₁-C₆ alkoxy group, among the above C₁-C₈ alkoxy groups in R¹, and of a carbonyl group, suitable specific examples of which include methoxycarbonyl group, ethoxycarbonyl group, and the like. A preferred group for X is a carboxyl group.

The "C₁-C₆ alkyl group" in Y of the above formula (I) means a C₁-C₆ linear or branched alkyl group, such as, for example, methyl, ethyl, n-propyl, n-butyl, n-pentyl, n-hexyl, isopropyl, isobutyl, sec-butyl, tert-butyl, isopentyl, neopentyl, tert-pentyl, isohexyl, 2-methylpentyl, and 1-ethylbutyl groups, suitable specific examples of which include methyl, ethyl, propyl, isopropyl groups, and the like. More preferred examples include a methyl group. Preferred groups for Y are C₁-C₆ alkyl groups, and a more preferred group is a methyl group.

Among the compounds represented by the formula (I), 2-[3-cyano-4-(2-methylpropoxy) phenyl]-4-methylthiazole-5-carboxylic acid is preferred.

Furthermore, it is possible that topiroxostat (4-[5-(pyridin-4-yl)-1H-1,2,4-triazol-3-yl]pyridine-2-carbonitrile) and the xanthine oxidase inhibitors described in International Publication No. WO2005/121153, International Publication No. WO2010/113942, or International Publication No. WO2007/043457 may also be used as compounds in the present invention.

Tumor lysis syndrome in the present invention is a metabolic disorder caused by the massive and abrupt release of cellular components into the circulating blood in the body when tumor cells are killed upon a therapy such as chemotherapy or radiotherapy, or when spontaneous lysis of tumors with high proliferative capacity occurs, and is defined as a fatal disease which causes hyperuricemia, hyperkalemia, hyperphosphatemia, hypocalcemia, and even acute renal failure and arrhythmia. In the 2008 American Society of Clinical Oncology (ASCO) guidelines, tumor lysis syndrome is defined in terms of diagnosis and severity by dividing it into laboratory TLS and clinical TLS. The former is defined as a case where there are 25% or more variations from baseline in any two or more of serum levels of phosphoric acid, calcium, potassium, and uric acid (Table 1), and the latter is defined by dividing it into grades 0 to 5 based on serum creatinine levels, severity of arrhythmia and seizure (Table 2).

**[Table 1]**

| Laboratory TLS Guidelines | | |
|---|---|---|
| Element | Measured Blood Levels | Variation from Baseline |
| Uric Acid | 8 mg/dL or more | 25% increase |
| Potassium | 6.0 mmol/L or more | 25% increase |
| Phosphoric acid | 2.1 mmol/L or more (children) | 25% increase |
| | 1.45 mmol/L or more (adults) | |
| Calcium | 1.75 mmol/L or less | 25% decrease |

**[Table 2]**

| Complication | 0 | 1 | 2 | 3 | 4 | 5 |
|---|---|---|---|---|---|---|
| Creatinine | 1.5 times or less | 1.5 | 1.5-3.0 | 3.0-6.0 | 6.0 times or more | Death |
| Cardiac arrhythmia | None | No intervention required | Drug therapy (no urgency) | Symptomatic (incompletely controlled by drug therapy or defibrillation) | Severe | Death |
| Seizure | None | None | Brief Transient | Reduced consciousness | Prolonged Repetitive (poorly controlled) | Death |

Prophylaxis of tumor lysis syndrome is considered important because once it has developed, it is difficult to treat as symptoms progress rapidly. For this reason, patients' risk of developing tumor lysis syndrome is determined, and prophylactic measures are taken before antitumor treatment is started. Such patients' risk of developing tumor lysis syndrome is defined as High Risk, Intermediate Risk, or Low Risk, depending on the type of tumor (Table 3). In addition, in the case where tumor lysis syndrome has already developed before antitumor therapy such as chemotherapy is performed and where hyperuricemia is presented and renal function is reduced, the patient is judged to have a risk corresponding to the risk of a patient at high risk for tumor lysis syndrome, as the risk of developing acute renal failure will further increase due to a therapy such as chemotherapy.

**[Table 3]**

| Type of Tumor | High Risk (High) | Intermediate Risk (Intermediate) | Low Risk (Low) |
|---|---|---|---|
| Non-Hodgkin's lymphoma | -Burkitt's -Lymphoblastic -Burkitt's acute lymphoblastic leukemia | Diffuse large B-cell lymphoma | Indolent non-Hodgkin's lymphoma |
| Acute lymphoblastic leukemia | WBC ≥100,000/µl | WBC 5,000-100,000/µl | WBC ≤50,000/µl |
| Acute myeloid leukemia | WBC ≥50,000/µl Monoblastic | WBC 10,000-50,000/µl | WBC ≤10,000/µl |
| Chronic lymphocytic lymphoma | | WBC 10,000-100,000/µl Treatment with fludarabine | WBC ≤10,000/µl |
| Other hematologic malignancies and solid cancers | | Rapid proliferation with expected rapid response to therapy | |

In the 2008 American Society of Clinical Oncology (ASCO) guidelines and the like, management with hydration plus rasburicase is recommended for high-risk patients, management with hydration plus allopurinol for intermediate-risk patients, or management with rasburicase for pediatric patients. In addition, even in intermediate-risk patients, management with rasburicase is recommended when hyperuricemia has already developed.

Good management of tumor lysis syndrome is generally defined as starting the administration of a uric acid-lowering agent prior to a therapy such as chemotherapy and radiotherapy, and continuing the administration until after the treatment of a tumor with chemotherapy, radiotherapy, etc., whereby at 48 hours after the start of administration of the uric acid-lowering agent, the blood uric acid levels reach 7.5 mg/dL or less if the patient is 13 years of age or older or 6.5 mg/dL or less if the patient is younger than 13 years of age, which is maintained until 24 hours after the end of administration of the uric acid-lowering agent. Further, the clinical significance of the management of tumor lysis syndrome is defined as inhibition of the development of tumor lysis syndrome as well as inhibition of the development of renal dysfunction such as acute renal failure associated with tumor lysis syndrome, or avoidance of dialysis.

That is, in the present invention, "normal level" is a target level for good management of tumor lysis syndrome, and means a condition in which serum uric acid levels are 7.5 mg/dL or less, and, with regard to renal function, estimated glomerular filtration rates are about 60 mL/min or more, or serum creatinine levels are about 1.1 mg/dL or less.

The compounds in the present invention are xanthine oxidase inhibitors, but are effective not only in intermediate-risk patients but also in high-risk patients, because their blood uric acid level-lowering effects yield larger reductions than those of the xanthine oxidase inhibitors used in conventional treatments for tumor lysis syndrome. Further, they are effective not only in patients who have not yet developed tumor lysis syndrome, but also in patients who have developed tumor lysis syndrome. Tumors in tumor lysis syndrome in the present invention include hematopoietic tumors, solid tumors, and the like.

Furthermore, the prophylactic or therapeutic agents of the present invention, even at a low dose, maintain blood uric acid levels within or lower them to within normal levels, prevent renal function from deteriorating or improve it, and have effects in preventing or treating tumor lysis syndrome. The prophylactic or therapeutic agents of the present invention can also maintain urinary excretion levels of uric acid within or lower them to within a normal range.

If necessary, the compounds represented by the above formula (I) can be converted into their pharmaceutically acceptable salts. Such salts include, for example, salts with inorganic acids, such as hydrochloric acid, hydrobromic acid, hydriodic acid, sulfuric acid, nitric acid, phosphoric acid, and carbonic acid; salts with organic acids, such as formic acid, acetic acid, propionic acid, trifluoroacetic acid, phthalic acid, oxalic acid, malonic acid, succinic acid, fumaric acid, maleic acid, lactic acid, malic acid, tartaric acid, citric acid, benzoic acid, methanesulfonic acid, ethanesulfonic acid, benzenesulfonic acid, and p-toluenesulfonic acid; salts with amino acids, such as lysine, arginine, ornithine, glutamic acid, and aspartic acid; salts with alkali metals, such as sodium, potassium, and lithium; salts with alkaline-earth metals, such as calcium and magnesium; salts with metals, such as aluminum, zinc, and iron; salts with organic bases, such as methylamine, ethylamine, t-octylamine, diethylamine, trimethylamine, triethylamine, ethylenediamine, piperidine, piperazine, pyridine, picoline, ethanolamine, diethanolamine, triethanolamine, cyclohexylamine, dicyclohexylamine, N-methyl glucamine, tris(hydroxymethyl)aminomethane, and N,N'-dibenzylethylenediamine; ammonium salts, and the like.

The active ingredient of the present invention can be used in any dosage form of solid preparation, semisolid preparation, liquid preparation, etc., and in any application preparation of oral formulations and parenteral formulations (injections, transdermal formulations, eye drops, suppositories, intranasal formulations, inhalants, and the like).

The therapeutic or prophylactic agent for tumor lysis syndrome of the present invention, comprising as an active ingredient a 2-phenylthiazole compound or a pharmaceutically acceptable salt thereof is prepared with carriers, excipients, and other additives commonly used to formulate pharmaceutical preparations. The carriers and excipients to formulate pharmaceutical preparations may be either solid or liquid and include, for example, lactose, magnesium stearate, starch, talc, gelatin, agar, pectin, Arabian gum, olive oil, sesame oil, cocoa butter, ethylene glycol, etc., and others that are commonly used. Administration may be in any form of oral administration such as via tablets, pills, capsules, granules, powders, or liquid preparations, or of parenteral administration such as via injections, such as intravenous injection and intramuscular injection, suppositories, or transdermal administration.

The dosage of the active ingredient of the present invention is an amount that is effective for the treatment or prevention of tumor lysis syndrome and can be determined depending on the symptom, age, weight of the patient, the type of concurrent treatment, the frequency of treatment, the nature of desired effect, the method of administration, or the like. Usually, the dosage is about 0.5 to 1000 mg per day, preferably, 10 to 120 mg for adults and 3 to 120 mg for children; for example, 3, 5, 10, 20, 30, 40, 50, 60, 80, or 120 mg per day is administered. The dose per administration is about 0.1 to 1000 mg, preferably 1 to 120 mg. For children, an amount that is appropriately reduced compared with that for adults is administered. Administration starts before or after disease development and may be performed daily or intermittently, usually 1 to 3 times/day, 1 to 7 days/week. It is preferred that the therapeutic or prophylactic agent of the present invention be prepared into formulations, such that these conditions can be met.

### [Examples]

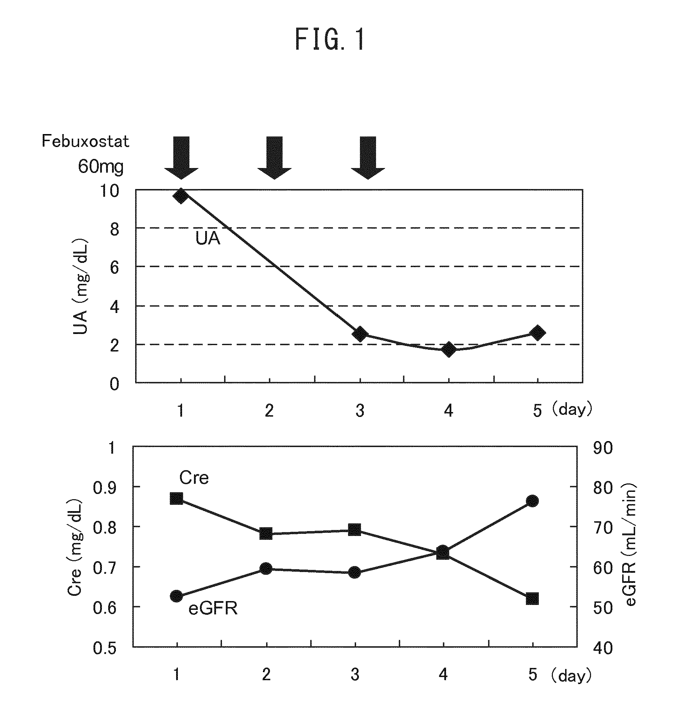

### Example 1. Investigation of the effects on serum uric acid levels and renal function in a tumor lysis syndrome patient (Figure 1)

In order to investigate the therapeutic effects of febuxostat on a high-risk tumor lysis syndrome patient, a high-risk patient with tumor lysis syndrome which had occurred without antitumor therapy, and already presented hyperuricemia, was given transfusions of fresh frozen plasma and packed red blood cells, and then in addition to increasing a urine volume by supplying fluid and to alkalizing urine, febuxostat (60 mg) was orally administered for 3 days, and the serum uric acid levels and renal function were compared with those before administration of febuxostat.

### <Subject Patient>

A 53-year-old female, Myelodysplastic syndrome (MDS), White blood cell count (WBC) 15400/µL

### <Results>

Serum uric acid level (UA): UA was 9.7 mg/dL on the day before the day from which febuxostat was administered, whereas on Day 4, after 3 days of the febuxostat administration (60 mg/day), it was lowered to 1.7 mg/dL indicating as high as an 82.4% reduction.

Serum creatinine (Cre) and estimated glomerular filtration rate (eGFR): Serum Cre was 0.87 mg/dL (estimated glomerular filtration rate: eGFR 53 mL/min) before administration of febuxostat, whereas on Day 4, after 3 days of the febuxostat administration (60 mg/day), the creatinine level was lowered to 0.73 mg/dL and eGFR was increased to 64 mL/min.

As shown in these results, by the administration of febuxostat, the serum uric acid level was rapidly lowered, and at the same time, renal function was also improved, which proved that this drug is effective for the treatment of tumor lysis syndrome.

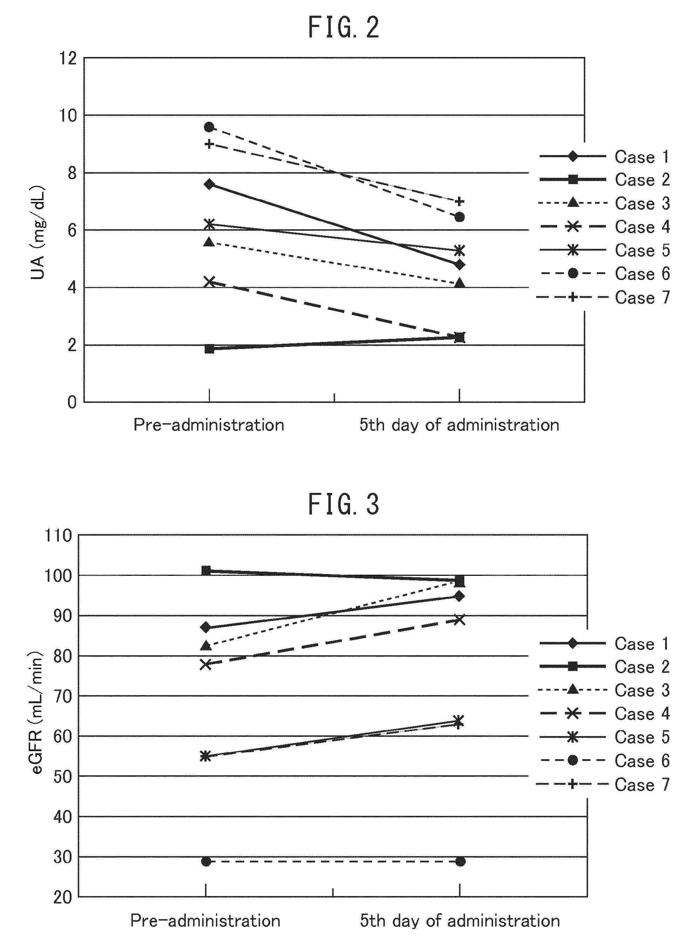

### Example 2. Investigation of the prophylactic effects on serum uric acid levels, renal function, and urinary excretion levels of uric acid in patients with hematological malignancies when chemotherapy was performed (Table 4, Figures 2 to 4)

In order to investigate the prophylactic effects of febuxostat on tumor lysis syndrome, 10 mg of febuxostat (the starting dose in gout and hyperuricemia) was orally administered once a day, daily, from just before or the day before chemotherapy was performed on patients with hematological malignancies at low or intermediate risk for tumor lysis syndrome, and the serum uric acid level and the renal function on the fifth day of administration were compared with those before the start of febuxostat administration. For cases in which urine collection was possible, the urinary excretion level of uric acid on the fifth day of administration was compared with that before the start of febuxostat administration.

### <Subject Patient (Table 4)>

Table 4 shows the age, sex, underlying disease, and white blood cell count of the patients with hematological malignancies.

Seven male and female cases (6 males and 1 female), aged 36 to 79 (median 70), acute myeloid leukemia (AML 3 cases), diffuse large B-cell lymphoma (DLBCL 2 cases), chronic myelomonocytic leukemia (CMML 1 case), chronic myeloid leukemia (CML 1 case), white blood cell count (WBC) 3,200 to 347,000/µL

**[Table 4]**

| Case | Age (years) | Sex | Underlying Disease | WBC (/µL) |
|---|---|---|---|---|
| 1 | 53 | Male | Diffuse large B-cell lymphoma (DLBCL) | 11,500 |
| 2 | 59 | Male | Acute myeloid leukemia (AML) | 25,400 |
| 3 | 74 | Male | Acute myeloid leukemia (AML) | 42,100 |
| 4 | 36 | Male | Acute myeloid leukemia (AML) | 3,200 |
| 5 | 76 | Male | Diffuse large B-cell lymphoma (DLBCL) | 5,800 |
| 6 | 79 | Female | Chronic myelomonocytic leukemia (CMML) | 60,700 |
| 7 | 70 | Male | Chronic myeloid leukemia (CML) | 347,000 |

### <Results>

Serum uric acid level (UA) (Figure 2): UA was 6.3mg/dL on average before the start of administration, whereas it was shown to be lowered to 4.6 mg/dL on average on the fifth day of febuxostat administration. Note that although an increase in UA was observed in Case 2, it was an increase from 1.9 mg/dL to 2.3 mg/dL; the UA level before administration was difficult to lower as it was low, but UA was maintained within normal levels.

Estimated glomerular filtration rate (eGFR) (Figure 3): eGFR was 69.7 mL/min on average before the start of administration, whereas it was increased to 76.9 mL/min on average on the fifth day of febuxostat administration. Note that although a reduction in eGFR was observed in Case 2, it was a reduction from 101 mL/min to 99 mL/min, and eGFR was maintained within normal levels. In the other cases, eGFR was able to be maintained or improved, and, in all cases, acute renal failure was prevented.

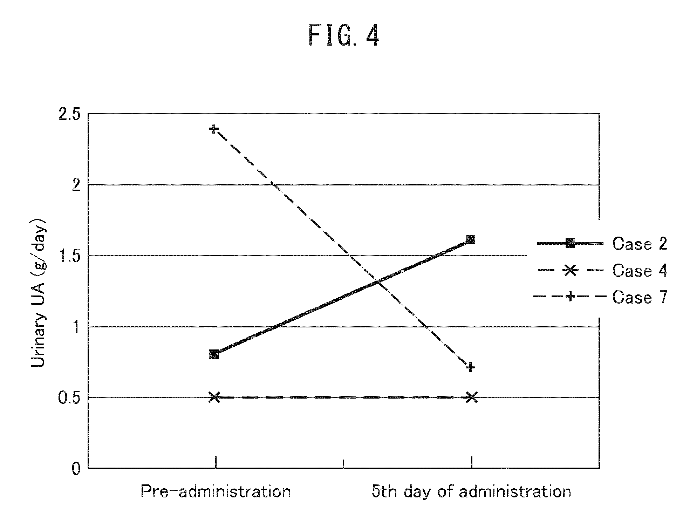

Urinary excretion level of uric acid (urinary UA) (Figure 4): For cases where urine collection was possible, urinary UA was 1.2 g/day on average before the start of administration, whereas it was lowered to 0.9 g/day on average on the fifth day of febuxostat administration. In one case, urinary UA was able to be maintained within normal levels, and, in a severe case in which WBC was high, urinary UA was lowered dramatically from 2.4 to 0.7 g/day in spite of the fact that chemotherapy was performed. Note that although an increase in urinary UA was observed in Case 2, it was only a slight increase from 0.8 g/day to 1.6 g/day.

As shown in these results, the prophylactic administration of a low dose of 10 mg of febuxostat was, in all cases, able to maintain the serum uric acid levels within or lower them to within normal levels in spite of the fact that chemotherapy was performed, and at the same time, also to improve renal function. Furthermore, in a particularly severe case, it was also able to dramatically lower the urinary excretion level of uric acid. Thus, this drug has proved effective for the prophylaxis of tumor lysis syndrome.

### Example 3. Investigation of the prophylactic effects on serum uric acid levels when chemotherapy is performed on malignant tumor patients with tumor lysis syndrome

In order to investigate prophylactic effects of febuxostat on tumor lysis syndrome, febuxostat is orally administered once a day, daily, from before chemotherapy is performed on a malignant tumor adult patient with tumor lysis syndrome, and an assessment is made by using serum uric acid levels as indicators. In addition, also for a malignant tumor pediatric patient with tumor lysis syndrome, similar investigation is conducted with febuxostat.

Results from these investigations prove that febuxostat is effective for the prevention of tumor lysis syndrome.

### [INDUSTRIAL APPLICABILITY]

The present invention can be used to treat or prevent tumor lysis syndrome.

## Claims

1. A therapeutic or prophylactic agent for tumor lysis syndrome, comprising as an active ingredient a 2-phenylthiazole compound represented by the following formula (I): 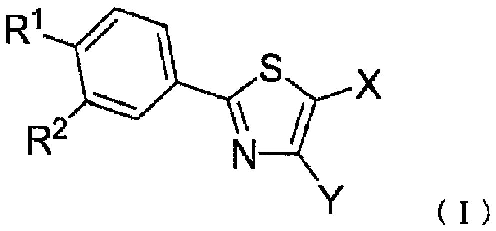 wherein:
R¹ represents a C₁-C₈ alkoxy group, a morpholino group, a 4-methylpiperazin-1-yl group, or a piperidino group;
R² represents a nitro group or a cyano group;
X represents a carboxyl group or a C₂-C₇ alkoxycarbonyl group; and
Y represents a hydrogen atom or a C₁-C₆ alkyl group;
or a pharmaceutically acceptable salt thereof.

2. The therapeutic or prophylactic agent according to Claim 1, wherein the tumor lysis syndrome is a tumor lysis syndrome in a high-risk patient.

3. The therapeutic or prophylactic agent according to Claim 1, wherein the tumor lysis syndrome is a tumor lysis syndrome in an intermediate-risk patient.

4. The therapeutic or prophylactic agent according to Claim 1, wherein the tumor lysis syndrome is a tumor lysis syndrome in a low-risk patient.

5. The therapeutic or prophylactic agent according to any one of Claims 1 to 4, wherein the tumor lysis syndrome is a tumor lysis syndrome involves hyperuricemia caused by chemotherapy or radiotherapy for malignant tumor.

6. The therapeutic or prophylactic agent according to any one of Claims 1 to 4, wherein the tumor lysis syndrome is a tumor lysis syndrome which occurs without being caused by antitumor therapy and in which hyperuricemia and reduced renal function are occurred.

7. The therapeutic or prophylactic agent according to any one of Claims 1 to 6, wherein the tumor lysis syndrome is a tumor lysis syndrome in a case where hyperuricemia is presented before implementation of antitumor therapy.

8. The therapeutic or prophylactic agent according to any one of Claims 1 to 7, wherein the tumor lysis syndrome is a tumor lysis syndrome in a case where renal function is reduced before implementation of antitumor therapy.

9. The therapeutic or prophylactic agent according to any one of Claims 1 to 8, **characterized in that** the 2-phenylthiazole compound represented by the formula (I), or a pharmaceutically acceptable salt thereof, is administered at 0.5 to 1000 mg per day, 1 to 7 days/week.

10. The therapeutic or prophylactic agent according to any one of Claims 1 to 9, which can lower blood uric acid levels following implementation of antitumor therapy to within normal levels or maintain them within the normal levels.

11. The therapeutic or prophylactic agent according to any one of Claims 1 to 10, which can improve renal function following implementation of antitumor therapy, compared with that before start of the antitumor therapy, or keep it normal.

12. The therapeutic or prophylactic agent according to any one of Claims 1 to 11, which can lower urinary excretion levels of uric acid following implementation of antitumor therapy to within normal levels or maintain them within the normal levels.

13. The therapeutic or prophylactic agent according to any one of Claims 1 to 12, wherein the 2-phenylthiazole compound represented by the above formula (I) is 2-[3-cyano-4-(2-methylpropoxy) phenyl]-4-methylthiazole-5-carboxylic acid or a pharmaceutically acceptable salt thereof.
